Europäisches Patentamt

European Patent Office

Office européen des brevets

(11) Publication number: **0 244 598**
**A1**

## (12) EUROPEAN PATENT APPLICATION

(21) Application number: **87103603.4**

(22) Date of filing: **12.03.87**

(51) Int. Cl.4: **C12N 15/00** , C12N 1/16 ,
//(C12N1/16,C12R1:84)

(30) Priority: **14.03.86 US 839845**

(43) Date of publication of application:
**11.11.87 Bulletin 87/46**

(84) Designated Contracting States:
**AT BE CH DE ES FR GB IT LI LU NL SE**

(71) Applicant: **PHILLIPS PETROLEUM COMPANY**
**5th and Keeler**
**Bartlesville Oklahoma 74004(US)**

(72) Inventor: **Buckholz, Richard Gordon**
**8933 Lombard Place 226**
**San Diego CA 92122(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) **Methanol and glucose responsive yeast regulatory regions.**

(57) Novel DNA fragments responsive to the presence of methanol and/or catabolite repressing carbon sources are disclosed, as well a methods for regulating protein expression therewith.

EP 0 244 598 A1

## METHANOL AND GLUCOSE RESPONSIVE YEAST REGULATORY REGIONS

This invention relates to recombinant DNA technology. In one of its aspects, the invention relates to methanol inducible regulatory regions. In another aspect, the invention relates to regulatory regions repressed in the presence of glucose. In yet another aspect, the invention relates to novel expression vectors incorporating regulatory regions of the invention, as well as novel organisms transformed therewith, and regulated polypeptide expression therewith.

## BACKGROUND

As recombinant DNA technology has developed in recent years, the controlled production by microorganisms of an enormous variety of useful polypeptides has become possible. Many eukaryotic polypeptides, such as for example, human growth hormone, leukocyte interferons, human insulin, human proinsulin, and the like, have already been produced by various microorganisms, The continued development of the field of recombinant DNA technology is expected in the future to yield even more effective means for producing useful polypeptide products using microorganisms. One desirable development would be the isolation and characterization of regulatory regions which are readily turned on and off by appropriate selection of media, growth conditions, or the like.

For example, the May, 1985 Molecular and Cellular Biology publication (pp. 1111-1121) by Ellis, Brust, Koutz, Waters, Harpold and Gineras discloses the isolation and characterization of DNA sequences which are responsive to the presence of methanol, and which in addition, are subject to catabolite repression in the presence of such catabolite repressing carbon sources as glucose. This invention is the result of further studies carried out on the regulatory regions of the above referenced disclosure.

## OBJECTS OF THE INVENTION

An object of the present invention, therefore, is the isolation and characterization of functional subfragments of the Ellis et. al. methanol responsive DNA sequences.

This and other objects of the invention will become apparent from the disclosure and claims herein provided.

## STATEMENT OF INVENTION

It has surprisingly been found that distinct subfragments of the regulatory regions disclosed by Ellis, Brust, Koutz, Waters, Harpold and Gingeras (Molecular and Cellular Biology, May, 1985, pp. 1111-1121) have unique regulatory properties; one such regulatory region being capable of full induction in the presence of methanol when carried in the host organism as a chromosomal element thereof; another such regulatory region being capable of full induction in the presence of methanol when carried in the host organism as an extrachromosomal element thereof; and yet another such regulatory region being capable of conferring upon heterologous promoter nucleotide sequences positioned downstream thereof the ability to be induced in the presence of methanol and repressed in the presence of catabolite repressing carbon sources.

## BRIEF DESCRIPTION OF THE FIGURES

Figure 1 is a restriction map of the region 5′ of the primary alcohol oxidase gene from *Pichia* (AOX1).
Figure 2 is a restriction map of plasmid pSAOH5.
Figure 3 is a restriction map of the yeast insert of plasmid pPG2.5. which is a pBR322 based plasmid.
Figure 4 is a restriction map of plasmid pBPf3l.
Figure 5 is a flow diagram of the vector constructions discussed in the Examples.
The following abbreviations are used in the figures to represent the restriction enzymes employed.

| Abbreviation | Restriction Enzyme |
|---|---|
| A | AvaII |
| B | BamHI |
| B$_2$ | BglII |
| Bc | BclI |
| H$_2$ | HincII |
| H$_3$ | HindIII |
| K | KpnII |
| Nd$_1$ | NdeI |
| Nr | NruI |
| Ps | PstI |
| Pv$_2$ | PvuII |
| R$_1$ | EcoRI |
| R$_5$ | EcoRV |
| S | SalI |
| Sm | SmaI |
| Ss | SstI |
| St | StuI |

In the attached figures, restriction sites employed for manipulation of DNA fragments, but which are destroyed upon ligation, are indicated by enclosing the abbreviation for the destroyed site in parenthesis.

## DETAILED DESCRIPTION OF THE INVENTION

In accordance with the present invention, there are provided novel DNA fragments comprising regulatory regions responsive to the presence of methanol in the culture medium with which a host organism for the DNA fragments is in contact. One of the novel fragments of the present invention is capable of promoting full induction in the presence of methanol when incorporated as a single copy into the genome of the host organism, i.e., when carried as a chromosomal element thereof, but not when carried as a extrachromosomal element. Another of the novel fragments of the present invention is capable of promoting full induction in the presence of methanol when carried as either an extrachromosomal element of the host organism, i.e., when maintained as an autonomous element in the host, or as a chromosomal element thereof. The regulatory regions of the DNA fragments of this invention are capable of controlling the transcription of messenger RNA when positioned at the 5′ end of the DNA which codes for the production of messenger RNA. Also included within the scope of the invention are mutants and functional equivalents of the above described DNA fragments.

The term "full induction" is employed in this disclosure to refer to the ability of a given DNA fragment to respond to the presence of methanol. The term relates to the ability of methanol responsive regulatory regions to induce the production of levels of protein product equivalent to the levels obtained using the 1 kilobase of non-coding sequences upstream of the wild-type AOX1 protein coding sequences in hosts transformed with in-phase regulatory region-structural gene constructs.

Further in accordance with the present invention, there is provided a DNA fragment comprising an upstream activator sequence which confers upon heterologous promoter nucleotide sequences positioned downstream thereof the ability for expression to be induced in the presence of methanol, and for expression to be repressed by the presence of a catabolite repressing carbon source in the culture medium with which a host organism for the DNA fragment is in contact. The regulatory region of the DNA of this embodiment of the invention is capable of controlling the transcription of messenger RNA when associated with a heterologous promoter positioned at the 5′ end of the DNA which codes for the production of messenger RNA. Also included within the scope of the invention are mutants and functional equivalents of the above described DNA fragment.

Still further in accordance with the present invention, plasmids and transformed organisms containing the above described DNA fragments are provided, as well as a process for producing polypeptides employing such transformed organisms.

In accordance with yet another embodiment of the present invention, a method is provided to control the responsiveness of methanol responsive regulatory regions by employing a variable number of nucleotides in the operable regulatory region. A range of methanol response can be achieved; varying from a nominal response (only a few percent of "full" induction), all the way up to full methanol induction.

The regulatory regions of the present invention are extremely useful for the regulated production of polypeptides using recombinant DNA modified hosts. For example, in one embodiment of this invention, methanol induced expression of polypeptide products from integrated expression cassettes is possible. In another embodiment, the methanol induced expression of polypeptide products from autonomous expression cassettes is possible.

In accordance with yet another embodiment, the expression of polypeptide products is controlled by transforming the host with a polypeptide coding sequence under the control of an upstream activator sequence, which is responsive to conditions of both methanol induction and catabolite repression; and thereafter maintaining the transformed host in the presence or absence of methanol or catabolite repressing carbon sources. Thus, in accordance with this specific embodiment, if a specified level of polypeptide products is desired, the composition of the culture medium can be adjusted until the desired level of polypeptide product is reached, and then further expression of the polypeptide coding sequence prevented by the addition to the culture medium of a catabolite repressing carbon source.

In accordance with this latter embodiment, the upstream activator sequence of the present invention is useful for achieving the regulated production of polypeptide products under the control of normally unregulated promoters. For example, constitutive promoters can be rendered responsive to methanol induction and catabolite repression by incorporation therein of the upstream activator sequences of the present invention. In this manner, the expression of any sequences which may function as a promoter of RNA transcription can be controlled by the presence or absence of methanol or catabolite repressing carbon sources.

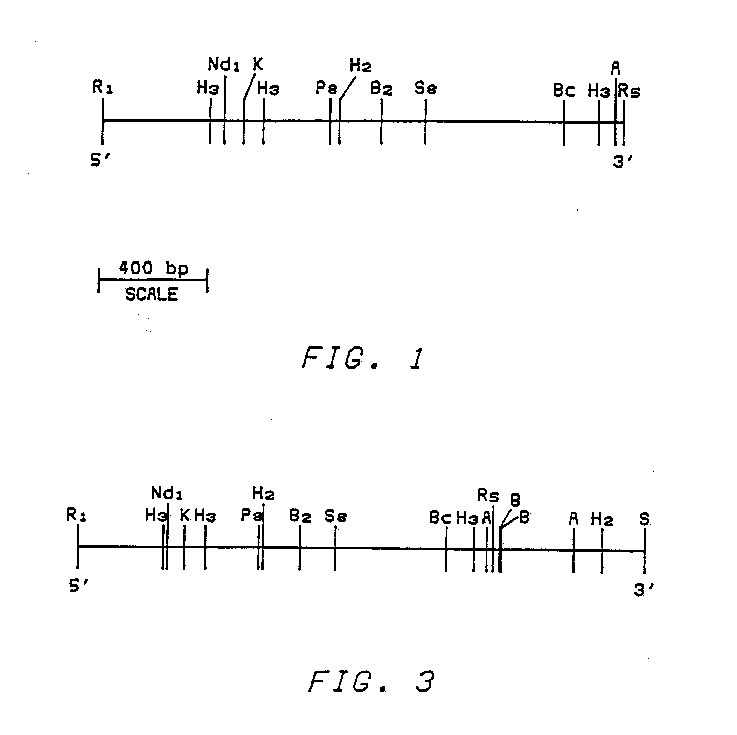

The approximately one kilobase pair (kbp) regulatory region obtained from the region 5′ of the primary alcohol oxidase gene (AOX1; see Figure 1) as disclosed by Ellis, Brust, Koutz, Waters, Harpold and Gingeras (Molecular and Cellular Biology, May, 1985, pp. 1111-1121) was subjected to BAL31 digestion from both the 3′ and 5′ ends, then the resulting mutagenized promoter fragments were fused with the E. coli lacZ gene, giving a series of vectors. The 5′ deletions gave the following vectors;

## Table I

| Vector | AO Promoter | Deleted Region |
|--------|-------------|----------------|
| pSAOH5 | -900→ATG | none |
| pBAZ3 | -709→ATG | 5′ 191 bp |
| pBAZ4 | -499→ATG | 5′ 401 bp |
| pBAZ6 | -372→ATG | 5′ 528 bp |
| pBAZ7 | -216→ATG | 5′ 684 bp |
| pBAZ8 | -197→ATG | 5′ 703 bp |

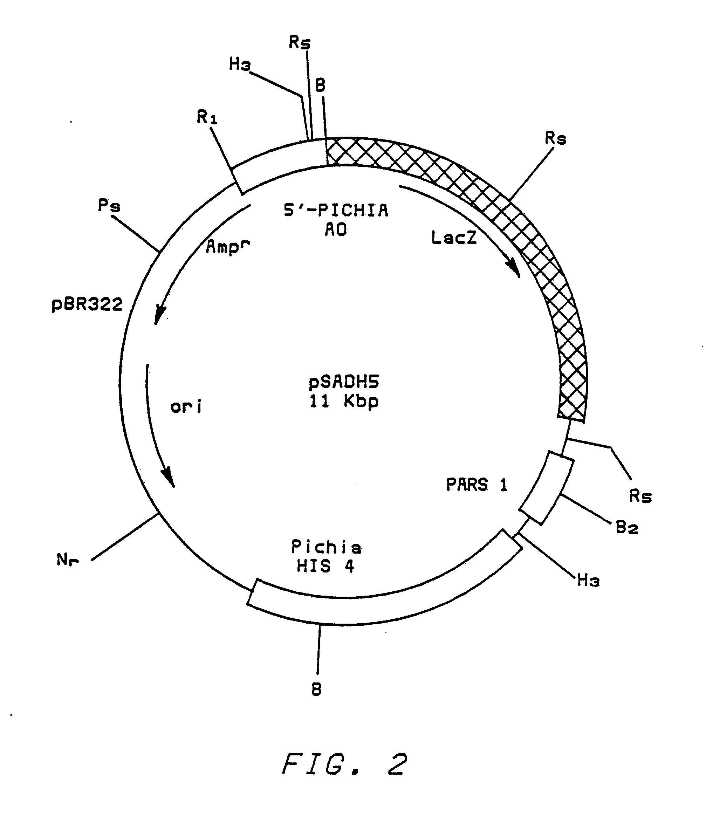

Vector pSAOH5 (see Figure 2) represents the intact 5′-alcohol oxidase regulatory region, while each of the vectors pBAZ3, 4, 6, 7 and 8 represent deletions from the 5′-end thereof. These vectors containing various regulatory region-structural gene (lacZ) constructs were used to transform a Pichia pastoris auxotrophic mutant and then assayed for β-galactosidase production when grown on methanol or glucose as carbon and energy source. While detailed analysis of the β-galactosidase expression results are presented in the Example, the results can be summarized here as follows:

(a) a weak methanol responsive region exists between 709 base pairs and 499 base pairs upstream to the ATG initiation codon for autonomous vectors containing these sequences, while full methanol response for integrated vectors does not require the sequence from 709 to 499 base pairs upstream. Thus,

4

while full methanol induction is obtained with the sequence 0-709 nucleotides for both integrated and autonomous vectors, the autonomous vectors display only about 50% methanol induction with the sequence 0-499 base pairs upstream of the alcohol oxidase ATG. The 709 base pair sequence required for full induction of expression with autonomous vectors is as follows:

## Sequence A

| | | | |
|---|---|---|---|
| 5'-GCTACTAACA | CCATGACTTT | ATTAGCCTGT | CTATCCTGGC |
| CCCCCTGGCG | AGGTCATGTT | TGTTTATTTC | CGAATGCAAC |
| AAGCTCCGCA | TTACACCCGA | ACATCACTCC | AGATGAGGGC |
| TTTCTGAGTG | TGGGGTCAAA | TAGTTTCATG | TTCCCAAATG |
| GCCCAAAACT | GACAGTTTAA | ACGCTGTCTT | GGAACCTAAT |
| ATGACAAAAG | CGTGATCTCA | TCCAAGATGA | ACTAAGTTTG |
| GTTCGTTGAA | ATGCTAACGG | CCAGTTGGTC | AAAAAGAAAC |
| TTCCAAAAGT | CGCCATACCG | TTTGTCTTGT | TTGGTATTGA |
| TTGACGAATG | CTCAAAAATA | ATCTCATTAA | TGCTTAGCGC |
| AGTCTCTCTA | TCGCTTCTGA | ACCCGGTGGC | ACCTGTGCCG |
| AAACGCAAAT | GGGGAAACAA | CCCGCTTTTT | GGATGATTAT |
| GCATTGTCCT | CCACATTGTA | TGCTTCCAAG | ATTCTGGTGG |
| GAATACTGCT | GATAGCCTAA | CGTTCATGAT | CAAAATTTAA |
| CTGTTCTAAC | CCCTACTTGG | ACAGGCAATA | TATAAACAGA |
| AGGAAGCTGC | CCTGTCTTAA | ACCTTTTTTT | TTATCATCAT |
| TATTAGCTTA | CTTTCATAAT | TGCGACTGGT | TCCAATTGAC |
| AAGCTTTTGA | TTTTAACGAC | TTTTAACGAC | AACTTGAGAA |
| GATCAAAAAA | CAACTAATTA | TTCGAAACG-3'. | |

At least nominal methanol induction is observed with as little as the first 197 nucleotides upstream of the ATG.

(b) a strong methanol responsive region lies between about 372 and 197 base pairs upstream of the ATG initiation codon, while a glucose responsive region lies between 372 and 216 base pairs upstream from the ATG initiation codon. This fragment has been shown by additional experiments, presented in greater detail in the Examples, to have the properties of an upstream activator sequence (UAS), i.e., it can confer upon downstream heterologous promoter sequences the ability to be induced by the presence of methanol and/or to be repressed by the presence of a catabolite repressing carbon source, such as glucose. This UAS fragment has the sequence:

## Sequence B

| | | | |
|---|---|---|---|
| | 5'-ATA | ATCTCATTAA | TGCTTAGCGC |
| AGTCTCTCTA | TCGCTTCTGA | ACCCGGTGGC | ACCTGTGCCG |
| AAACGCAAAT | GGGGAAACAA | CCCGCTTTTT | GGATGATTAT |
| GCATTGTCCT | CCACATTGTA | TGCTTCCAAG | ATTCTGGTGG |
| GAATACTGCT | GATAGCCTAA | CGTTCATGAT | CAA-3' |

A series of deletions from the 3'-end of the 5'-AOX1 promoter was prepared, then subcloned into the 5' deletion vectors pBAZ6 and pBAZ8, giving the sequence of vectors listed in Table II.

5

## Table II

| Vector | Deletion |
|---|---|
| pBAZ801 | -264→197 |
| pBAZ802 | -347→197 |
| pBAZ803 | -445→197 |
| pBAZ805 | -532→197 |
| pBAZ804 | -694→197 |
| pBAZ604 | -445→372 |
| pBAZ601 | -532→372 |
| pBAZ603 | -542→372 |
| pBAZ602 | -694→372 |

The set of vectors listed in Table II contain variable internal deletions between base positions 694 and 197 relative to the ATG initiation codon of AOX1 . The 5′ portion of the promoter sequences contained in the vectors summarized in Table II are derived from the 3′ end deletions of the alcohol oxidase regulatory region, while the 3′ portion of the regulatory regions contained in the vectors summarized in Table II are derived from the 5′ end deletions from the alcohol oxidase regulatory region, pBAZ6 and pBAZ8. As before, this regulatory region-β-galactosidase structural gene construct collection was used to transform a Pichia pastoris auxotrophic mutant, then assayed for β-galactosidase production when grown on methanol or glucose. While detailed analyses of the assay results are presented in the Examples, the results can be summarized by stating that a very strong methanol inducible element lies in an approximately 100 base pair region between about 260 and 370 nucleotides upstream from the ATC initiation codon. In addition, a weaker methanol regulatable DNA fragment is also found in the region from about 500 up to 710 nucleotides upstream from the ATG initiation codon. Furthermore, a glucose repressible region is observed in the region from about 350 to 375 base pairs upstream from the alcohol oxidase ATG initiation codon. This glucose repressible region is directly upstream of the methanol inducible region, and may overlap with the methanol responsive sequences.

Either of the methanol inducible elements identified above can be inserted, alone or in combination, into expression vectors to enable methanol induced gene expression.

Conversely, the glucose repressible region identified above can be deleted from methanol inducible promoter elements, thus enabling nonrepressed gene expression when a modified promoter is used in expression vectors and the resulting transformed organisms are grown in the presence of glucose. Alternatively, the glucose repressible regions can be purposely included in specially designed vectors in order to enable rapid "on-off" control of gene expression.

In accordance with another embodiment of the present invention, there is provided a method for controlling the responsiveness of an integrated regulatory region in the presence of methanol by employing as the regulatory region a nucleotide sequence having varying number of base pairs. Thus, with a minimum of the following 197 base pairs, about 5% of full methanol induction is obtained:

## Sequence C

5′-AAATTTAA

| CTGTTCTAAC | CCCTACTTGG | ACAGGCAATA | TATAAACAGA |
|---|---|---|---|
| AGGAAGCTGC | CCTGTCTTAA | ACCTTTTTTT | TTATCATCAT |
| TATTAGCTTA | CTTTCATAAT | TGCGACTGGT | TCCAATTGAC |
| AAGCTTTTGA | TTTTAACGAC | TTTTAACGAC | AACTTGAGAA |
| GATCAAAAAA | CAACTAATTA | TTCGAAACG-3′. | |

Essentially full methanol induction is observed with integrated expression vectors when all of the following added sequence is employed:

## Sequence D

```
                    5'-ATA      ATCTCATTAA    TGCTTAGCGC
AGTCTCTCTA    TCGCTTCTGA    ACCCGGTGGC    ACCTGTGCCG
AAACGCAAAT    GGGGAAACAA    CCCGCTTTTT    GGATGATTAT
GCATTGTCCT    CCACATTGTA    TGCTTCCAAG    ATTCTGGTGG
GAATACTGCT    GATAGCCTAA    CGTTCATGAT    CAA-3'.
```

The approximate degree of methanol induction as a function of the size of the regulatory region employed in integrated expression vectors is set forth in Table III:

## Table III

| Nucleotide Sequence | % Methanol Induction |
|---|---|
| 0-197 | 5 |
| 0-197 plus 347-372 | 5 |
| 0-197 plus 264-372 | 25 |
| 0-372 | 100 |

Similarly, a method is also provided for controlling the responsiveness of an autonomous regulatory region to the presence of methanol by employing as the regulatory region a nucleotide sequence having at least the 197 nucleotides set forth in Sequence C above, with the following added sequence required to achieve full methanol expression:

## Sequence E

```
5'-GCTACTAACA    CCATGACTTT    ATTAGCCTGT    CTATCCTGGC
CCCCCTGGCG    AGGTCATGTT    TGTTTATTTC    CGAATGCAAC
AAGCTCCGCA    TTACACCCGA    ACATCACTCC    AGATGAGGGC
TTTCTGAGTG    TGGGGTCAAA    TAGTTTCATG    TTCCCAAATG
GCCCAAAACT    GACAGTTTAA    ACGCTGTCTT    GGAACCTAAT
ATGACAAAAG    CGTGATCTCA    TCCAAGATGA    ACTAAGTTTG
GTTCGTTGAA    ATGCTAACGG    CCAGTTGGTC    AAAAAGAAAC
TTCCAAAAGT    CGCCATACCG    TTTGTCTTGT    TTGGTATTGA
TTGACGAATG    CTCAAAAATA    ATCTCATTAA    TGCTTAGCGC
AGTCTCTCTA    TCGCTTCTGA    ACCCGGTGGC    ACCTGTGCCG
AAACGCAAAT    GGGGAAACAA    CCCGCTTTTT    GGATGATTAT
GCATTGTCCT    CCACATTGTA    TGCTTCCAAG    ATTCTGGTGG
GAATACTGCT    GATAGCCTAA    CGTTCATGAT    CAA-3'.
```

The approximate degree of methanol induction as a function of the size of the regulatory region employed is set forth in Table IV:

Table IV

| Nucleotide Sequence | % Methanol Induction |
|---|---|
| 0-197 | 4 |
| 0-216 | 13 |
| 0-372 | 50 |
| 0-499 | 50 |
| 0-709 | 100 |

In accordance with one embodiment of the present invention, the upstream activator sequence (UAS) set forth in Sequence B was inserted upstream of *Pichia* sequences which are ordinarily insensitive to the presence of glucose and/or methanol. When grown on glucose, the expression of the gene product ($\beta$-galactosidase) by the UAS-containing transformant was repressed by a factor of more than 400 with respect to the non-UAS containing construct pBPf3I. This repression was alleviated by growth in methanol; the level of $\beta$-galactosidase production was increased over 170-fold above that of the glucose-grown transformant:

Table V

| Insert in pBPf3I | Ratio of $\beta$-galactosidase produced when grown on methanol/glucose |
|---|---|
| None | 1.07 |
| -197 to -372 | 174 |

The specific transformants, results, etc. are described in detail in the Examples.

The invention will now be described in greater detail with reference to the following nonlimiting examples.

8

## EXAMPLES

The buffers and solutions employed in the following examples have the compositions given below:

```
SD plates          2% agar
                   6.7 g yeast nitrogen base without amino acids
                      (DIFCO)
                   2 wt. % dextrose
                   in 1L of water

YNB broth        - 6.7 g yeast nitrogen base without amino acids
                      (DIFCO)
                   in 1L of water

Z buffer         - 0.1M sodium phosphate, pH 7.0
                   0.01M KCl
                   0.001M MgSO₄
                   0.039M β-mercaptoethanol

X-gal indicator plates
                 - 2% agar
                   0.5 vol. % methanol
                   6.7 g yeast nitrogen base without amino acids
                      (DIFCO)
                   0.4 mg biotin
                   40 mg X-gal (5-bromo-4-chloro-3-indolyl-β-D-
                      galactoside)
                   in 1 L water,
                   adjusted to pH7 with monobasic potassium
                      phosphate
              -- EGTA= ethyleneglycol-bis-(B-aminoethyl ether)-N,N,N',N'-
                   tetra-acetic acid (Sigma Co.)
```

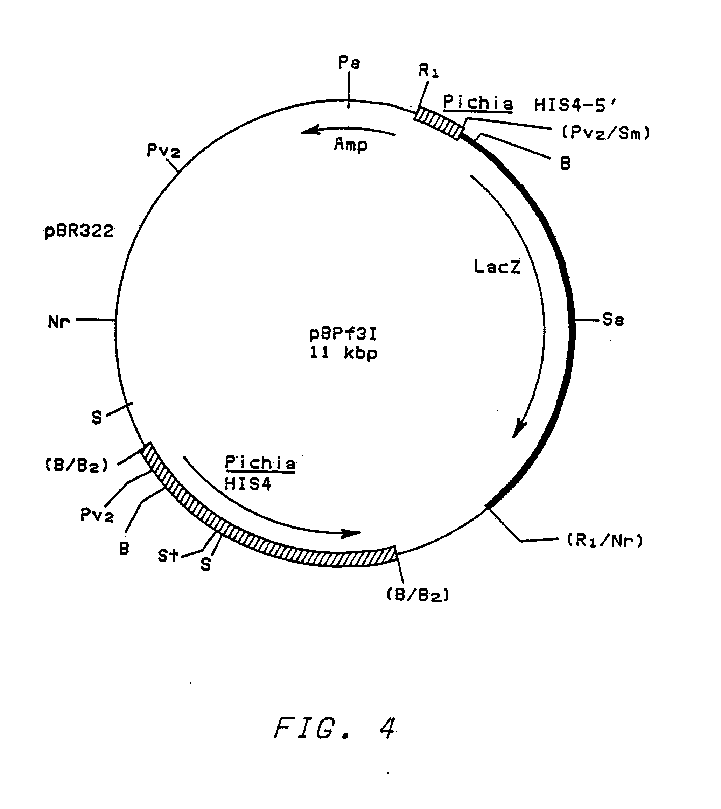

The strains employed in the following examples are available from recognized depositories under the accession numbers given below: --bacterial strains JM103 , HB101, and MC1061 are commonly available.
--*Pichia* GS115 = NRRL Y-15851
--pSAOH5 (transformed into GS115 = NRRL Y-15853; transformed into *E. coli* available as NRRL B-15862)
--pBPf3I is derived from plasmid pBPf1 (accession number NRRL B-15892 by the following sequence of steps: The 0.6 kbp EcoRI-PvuII fragment from pYJ8 (accession number NRRL B-15889),which fragment contains the *Pichia*HIS 4 promoter sequences, was inserted into the EcoRI-SmaI sites of pBPf1. The resultant plasmid was designated pBPf3. Plasmid pBPf3 was digested with PstI and NruI to release the fragment containing the *lacZ* sequences. This fragment was ligated with the Pst I-EcoRV fragment of pYM4 (which contains the HIS 4-containing BglII fragment of pYJ8 inserted into the BamHI site of pBR322) to produce plasmid pBPf3I, illustrated in Figure 4.
--pPG2.5 (derivable from pPG4.0 = NRRL B-15868 as detailed below)

9

### Example I

Construction of 5'-end deletions of AOX1 promoter

Thirty micrograms of plasmid pSAOH5 (Figure 2) were digested to completion with EcoRI, phenol-extracted, and ethanol-precipitated. The dried DNA pellet was dissolved in water (final reaction volume = 100 μL) and digested at 23°C with 1.2 units of BAL31. Ten-microliter aliquots were removed, at three-minute intervals, to 10 μL of 40 mM EGTA, phenol-extracted, and ethanol-precipitated.

The redissolved DNA pellet from each aliquot was treated with 1 unit on Klenow DNA polymerase, to enhance blunt ends, ligated to EcoRI linkers (5'-GGAATTCC-3'), and digested to completion with PstI and EcoRI to release the approximately 9.0 kbp vector fragment. The PstI-EcoRI-digested DNA was then ligated to the 750 bp Pst I-EcoRI fragment from pBR322 in order to regenerate the β-lactamase gene. The ligated DNA was transformed into JM103 and transformants selected for ampicillin resistance. Individual transformants, derived from each of the BAL31-digested aliquots of pSAOH5 and which tested positive for β-galactosidase on X-gal indicator plates, were picked and examined by the mini DNA preparation procedure for the presence of a plasmid containing a novel EcoRI restriction site in the AXO1 promoter. Digestion to completion with a mixture of Eco RI and EcoRV (see Figure 2) allowed the identification of clones containing various lengths of the AXO1 promoter fused to the E. coli lacZ gene. The designation of these clones is shown below:

## Table I

| Vector | AO Promoter | Deleted Region |
|--------|-------------|----------------|
| pSAOH5 | −900→ATG | none |
| pBAZ3 | −709→ATG | 5' 191 bp |
| pBAZ4 | −499→ATG | 5' 401 bp |
| pBAZ6 | −372→ATG | 5' 528 bp |
| pBAZ7 | −216→ATG | 5' 684 bp |
| pBAZ8 | −197→ATG | 5' 703 bp |

Plasmids containing these shortened AXO1 promoter sequences were then used to transform P. Pastoris strain GS115 employing published procedures (Cregg et. al., Molecular and Cellular Biology 5. 3376-3385 (1985)) and transformants were selected for His+ phenotype. His+ transformants were grown on various carbon sources and assayed for β-galactosidase production (see Table VI).

## Table VI

| | | β-Galactosidase, Units/OD$_{600}$ | |
|---|---|---|---|
| | | Autonomous Vector | |
| Vector | Deletion | Glucose Grown | Methanol Grown |
| pSAOH5 | None | 1 | 1000 |
| pBAZ3 | 5' (191 bp) | 1 | 1000 |
| pBAZ4 | 5' (401 bp) | 1 | 500 |
| pBAZ6 | 5' (528 bp) | 1 | 500 |
| pBAZ7 | 5' (684 bp) | 15 | 150 |
| pBAZ8 | 5' (703 bp) | 15 | 40 |

Plasmids pBAZ6 and pBAZ8 were chosen for further study based on the indication that pBAZ6 contained the majority of the methanol inducible-glucose repressible sequences and pBAZ8 was mostly deleted of these responsive areas.

## Example II

Construction of 3'-end deletions of AOX1 promoter

Eighteen micrograms of plasmid pPG2.5 (a pBR322-based plasmid containing the approximately 2.5 kbp EcoRI-SalI fragment of pPG4.0 shown in Figure 3) were digested to completion with BamHI, phenol-extracted, and ethanol-precipitated. The dissolved DNA pellet (final reaction volume = 100 $\mu$l) was digested at 23°C with 1.2 units of BAL31, and 10-microliter aliquots were removed at three-minute intervals to 10 microliters of 40 mM EGTA, phenol-extracted, and ethanol-precipitated.

The redissolved DNA pellet from each aliquot was treated with 1 unit of Klenow DNA polymerase to enhance blunt ends, ligated to EcoRI linkers, and used to transform E. coli strain HB101 to ampicillin resistance. Individual transformants derived from each of the BAL31-digested aliquots of pPG2.5 were picked and examined by the mini DNA preparation procedure for the presence of a plasmid containing a novel EcoRI restriction site in the AOX1 promoter.

Plasmids containing a novel EcoRI restriction site located at a position in the AOX1 promoter corresponding to 264, 347, 445, 532, 542 and 694 nucleotides upstream of the AOX1 ATG were isolated and designated pPG2.5 $\Delta$4, $\Delta$5, $\Delta$7, $\Delta$10, $\Delta$12, and $\Delta$11, respectively, then digested withEcoRI. These digestions produced novel Eco RI fragments of approximately 1186, 1103, 1105, 918, 908, and 756 base pairs, respectively. The resultingEcoRI fragments, which contained the portion of the AOX1 promoter located upstream of the EcoRI linker inserted subsequent to BAL31 mutagenesis, were isolated and ethanol-precipitated.

## Example III

Growth of Pichia Yeasts Transformed with lacZ Fusion Plasmids

A transformed colony was picked and streaked on a SD plate. A single colony from the streak was inoculated into 15 mL of YNB broth + 2% glycerol in a 50 mL shake flask and shaken at 30°C at 250 rpm overnight. The $OD_{600}$ of the culture 24 hours later was between 2-3.

For YNB-methanol growth, 1 $OD_{600}$ of culture was withdrawn from the shake flask and centrifuged in an IEC centrifuge for seven minutes at 2000 x g at room temperature. The pelleted cells were washed once with sterile water, and resuspended in 20 mL of YNB broth + 0.5% methanol in a 50 mL shake flask ($OD_{600}$ = 0.05). The culture was incubated at 30°C at 250 rpm for 16-20 hours, at which time the $OD_{600}$ was between 0.3-0.5.

For YNB-glucose growth, 0.2 $OD_{600}$ of culture was withdrawn from the glycerol shake flask and inoculated directly into 20 mL of YNB broth + 2% glucose in a 50mL shake flask ($OD_{600}$ = 0.01). The culture was incubated at 30°C at 250 rpm for 16-20 hours, at which time the $OD_{600}$ was between 0.3-0.5.

## Example IV

B-galactosidase Assay of Pichia Yeasts Grown on Glucose or Methanol Media

For assay of methanol grown cells, a volume of culture corresponding to an $OD_{600}$ = 0.1 was withdrawn and centrifuged in a DYNAC clinical centrifuge at top speed (3350 rpm) for five minutes at room temperature. For assay of glucose grown cells, a volume of culture corresponding to an $OD_{600}$ = 0.5 was withdrawn and centrifuged as above.

The cell pellets were washed once with water, and resuspended in 1 mL of Z buffer. The resulting suspension was mixed with 30 $\mu$l of $CHCl_3$ and 30 $\mu$l of 0.1% SDS, mixed by vortexing and incubated at 30°C for five minutes.

The reaction was initiated by the addition of 200 $\mu$l of prewarmed O-nitrophenyl-B-D-galactopyranoside (4 mg/mL) (SIGMA Co.) and mixed by vortexing. The reaction was incubated for 2-20 minutes until the appearance of a visible yellow color. The reaction was terminated by the addition of 0.5 mL of a 1.0 M solution of $Na_2CO_3$, the cells pelleted, and the $OD_{420}$ of the supernatant determined. Units of $\beta$-galactosidase were calculated as:

$$1 \text{ unit} = \frac{(378)(A^{420})}{\text{time of incubation}}$$

Example V

A. Construction of autonomous plasmids pBAZ801-805 and pBAZ601-604

Plasmids pBAZ6 and pBAZ8 were digested to completion with EcoRI, ligated to the Eco RI, fragments isolated in Example II above, and used to transform *E. coli* strain HB101 to ampicillin resistance. Transformants were analyzed by the mini DNA preparation procedure for the presence of the EcoRI fragment, then digested with the PstI to ascertain that the fragment orientation in the plasmid corresponded to that of pPG2.5. Plasmid designations for the ligation products of pBAZ8 and pBAZ6 containing EcoRI fragments in the proper orientation are listed in Table VII, below.

## TABLE VII

| | Vector | EcoRI Fragment Size | Deletion |
|---|---|---|---|
| pBAZ | 801 | 1186 | -264->197 |
| | 802 | 1103 | -347->197 |
| | 803 | 1005 | -445->197 |
| | 805 | 918 | -532->197 |
| | 804 | 756 | -694->197 |
| | 604 | 1005 | -445->372 |
| | 601 | 918 | -532->372 |
| | 603 | 908 | -542->372 |
| | 602 | 756 | -694->372 |

The plasmids were transformed into *P. pastoris* strain GS115; His+ transformants were grown on YNB-glucose or YNB-methanol medial and assayed for $\beta$-galactosidase. The assay results are presented in Table VIII.

## Table VIII

| | | $\beta$-Galactosidase, Units/OD$_{600}$ Autonomous Vector | |
|---|---|---|---|
| Vector | Deletion | Glucose Grown | Methanol Grown |
| pSAOH5 | None | 1 | 1000 |
| pBAZ801 | 197-264 | 3 | 500 |
| pBAZ802 | 197-347 | 1 | 150 |
| pBAZ803 | 197-445 | 1 | 140 |
| pBAZ805 | 197-532 | 1 | 150 |
| pBAZ804 | 197-694 | 10 | 100 |
| pBAZ604 | 372-445 | 1 | 1000 |
| pBAZ601 | 372-532 | 1 | 500 |
| pBAZ603 | 372-542 | 1 | 800 |
| pBAZ602 | 372-694 | 1 | 600 |

Based on the observation that the deletion in pBAZ801 reduced expression 50% from wild type, while that in pBAZ802 reduced expression to only 15% of wild type, it was concluded that the sequences between -372 and -197 contain a weak methanol responsive region between -264 and -197 and a strong methanol responsive region between -347 and -264. Furthermore, a significant extent of the glucose repression sequences lie upstream of -347.

B. Construction of integrated plasmids pBAZ801I-805I and pBAZ601I-604I

Plasmids pBAZ6 and pBAZ8 were digested to completion with BgIII, treated with Klenow DNA polymerase to destroy the BGIII site, recircularized with ligase and used to transform E. coli strain HB101 to ampicillin resistance. The resultant plasmids pBAZ∆BGI II and pBAZ8∆BgIII, were digested to completion with EcoRI, ligated to the EcoRI fragments isolated in Example II, and used to transform E. coli strain HB101 to ampicillin resistance. Transformants were analyzed by the mini DNA preparation procedure for the presence of the novel EcoRI fragment, then digested with PstI to ascertain that the fragment orientation in the plasmid corresponded to that of pPG2.5.

Plasmid designations for ligation products of pBAZ8 ∆BgIII and pBAZ6 ∆BGIII containing EcoRI fragments in the proper orientation are listed in Table IX.

## TABLE IX

| | Vector | EcoRI Fragment Size | Deletion |
|---|---|---|---|
| pBAZ | 801I | 1186 | -264->197 |
| | 802I | 1103 | -347->197 |
| | 803I | 1005 | -445->197 |
| | 805I | 918 | -532->197 |
| | 804I | 756 | -694->197 |
| | 604I | 1005 | -445->372 |
| | 601I | 918 | -532->372 |
| | 603I | 908 | -542->372 |
| | 602I | 756 | -694->372 |

The plasmids were digested with BgIII to direct them to the AOXI locus, and transformed into P. pastoris strain GS115. Stable HIS $^+$ transformants were isolated, grown on YNB-glucose or YNB-methanol media, and assayed for β-galactosidase. The assay results are presented in Table X.

## Table X

| Vector | Deletion | β-Galactosidase, Units/OD$_{600}$ Integrated Vector | |
|---|---|---|---|
| | | Glucose Grown | Methanol Grown |
| pSAOH5 | None | 1 | 320 |
| pBAZ801I | 197-264 | 1 | 73 |
| pBAZ802I | 197-347 | 1 | 19 |
| pBAZ803I | 197-445 | 1 | 30 |
| pBAZ805I | 197-532 | 1 | 20 |
| pBAZ804I | 197-694 | ND | ND |
| pBAZ604I | 372-445 | 1 | 290 |
| pBAZ601I | 372-532 | 1 | 300 |
| pBAZ603I | 372-542 | 0 | 250 |
| pBAZ602I | 372-694 | 1 | 320 |

The stability of the integration event allowed the conclusion that sequences upstream of -372 are not required for methanol induction for integrated vectors, since pBAZ602I produced wild type levels of $\beta$-galactosidase. In addition, the results confirm that strong and weak methanol responsive regions lie between -347 and -264 and -197, respectively.

Example VI

The $\beta$-galactosidase expression results with the various 5' deletions and internal deletion expression cassettes in both the autonomous and integrated form are presented in Table XI for ease of comparison.

## Table XI

| | | $\beta$-Galactosidase, Units/OD$_{600}$ | | | |
| | | Autonomous Vector | | Integrated Vector | |
| Vector | Deletion | Glucose Grown | Methanol Grown | Glucose Grown | Methanol Grown |
|---|---|---|---|---|---|
| pSAOH5 | None | 1 | 1000 | 1 | 320 |
| pBAZ3 | 5' (191 bp) | 1 | 1000 | | |
| pBAZ4 | 5' (401 bp) | 1 | 500 | | |
| pBAZ6 | 5' (528 bp) | 1 | 500 | | |
| pBAZ7 | 5' (684 bp) | 15 | 150 | | |
| pBAZ8 | 5' (703 bp) | 15 | 40 | | |
| pBAZ801,801I | 197-264 | 3 | 500 | 1 | 73 |
| pBAZ802,802I | 197-347 | 1 | 150 | 1 | 19 |
| pBAZ803,803I | 197-445 | 1 | 140 | 1 | 30 |
| pBAZ805,805I | 197-532 | 1 | 150 | 1 | 20 |
| pBAZ804,804I | 197-694 | 10 | 100 | ND | ND |
| pBAZ604,604I | 372-445 | 1 | 1000 | 1 | 290 |
| pBAZ601,601I | 372-532 | 1 | 500 | 1 | 300 |
| pBAZ603,603I | 372-542 | 1 | 800 | 0 | 250 |
| pBAZ602,602I | 372 694 | 1 | 600 | 1 | 320 |

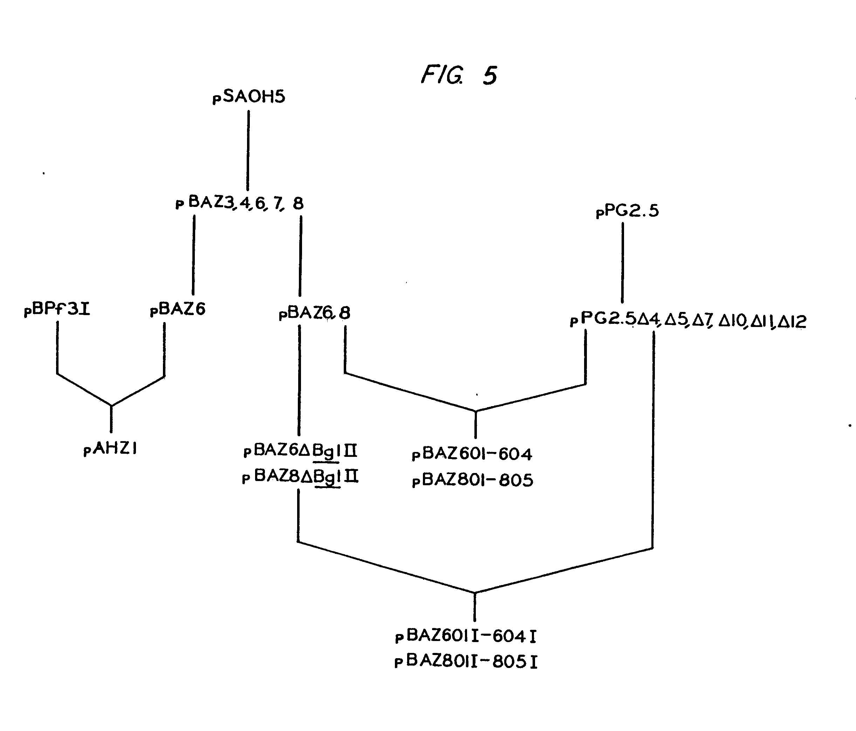

The derivation of each of these vectors is summarized in schematic fashion in Figure 5.

Example VII

Construction of pAHZ1

Plasmid pBAZ6 was digested with EcoRI, ligated to EcoRI-BglII synthetic adaptors (5'-AATTAGATCT-3'), and digested to completion with BGlII and BclI. The resultant ~180 bp fragment containing the AOX1 UAS was isolated and ligated into the unique BGlII site of pBPf3I (Figure 4), and the resulting ligation products were used to transform E. coli strain MC1061 to ampicillin resistance. A transformant containing a plasmid with a single UAS insert was identified by digestion with BamHI; this plasmid was designated pAHZ1.

Plasmid pAHZ1 was digested with StuI and used to transform P. pastoris GS115; stable HIS⁺ transformants were isolated, grown on YNB-glucose and YNB-methanol media, and assayed for $\beta$-galactosidase production. The results of this analysis are shown below:

14

## Table XII

## β-gal, Units/OD$_{600}$

| plasmid | glucose grown | methanol grown |
|---------|---------------|----------------|
| pBPf3I  | 49.8          | 53.5           |
| pAHZ1   | 0.1           | 17.4           |

These data show that the 175 base pairs contained between -372 and -197 of the AOX1 promoter on BglII-BclI DNA fragment are not only necessary but also sufficient to confer methanol induction and glucose repression of the *Pichia HIS* 4 gene promoter sequences, and define this region as containing an Upstream Activator Sequence.

The examples have been provided merely to illustrate the practice of the invention and should not be read so as to limit the scope of the invention or the appended claims in any way. Reasonable variations and modifications, not departing from the essence and spirit of the invention, are contemplated to be within the scope of patent protection desired and sought.

The following part of the description are preferred embodiments 1 to 33.

On pages 23 - 29 of the description, instead of "claim(s) read "embodiment(s)" each.

1. A DNA fragment responsive to the presence of methanol in the culture medium in which the host bearing said fragment is grown; wherein said fragment is maintained as an autonomous element in said host; and wherein said fragment comprises at least the nucleotide sequence:

```
5'-GCTACTAACA   CCATGACTTT   ATTAGCCTGT   CTATCCTGGC
   CCCCCTGGCG   AGGTCATGTT   TGTTTATTTC   CGAATGCAAC
   AAGCTCCGCA   TTACACCCGA   ACATCACTCC   AGATGAGGGC
   TTTCTGAGTG   TGGGGTCAAA   TAGTTTCATG   TTCCCAAATG
   GCCCAAAACT   GACAGTTTAA   ACGCTGTCTT   GGAACCTAAT
   ATGACAAAAG   CGTGATCTCA   TCCAAGATGA   ACTAAGTTTG
   GTTCGTTGAA   ATGCTAACGG   CCAGTTGGTC   AAAAAGAAAC
   TTCCAAAAGT   CGCCATACCG   TTTGTCTTGT   TTGGTATTGA
   TTGACGAATG   CTCAAAAATA   ATCTCATTAA   TGCTTAGCGC
   AGTCTCTCTA   TCGCTTCTGA   ACCCGGTGGC   ACCTGTGCCG
   AAACGCAAAT   GGGGAAACAA   CCCGCTTTTT   GGATGATTAT
   GCATTGTCCT   CCACATTGTA   TGCTTCCAAG   ATTCTGGTGG
   GAATACTGCT   GATAGCCTAA   CGTTCATGAT   CAAAATTTAA
   CTGTTCTAAC   CCCTACTTGG   ACAGGCAATA   TATAAACAGA
   AGGAAGCTGC   CCTGTCTTAA   ACCTTTTTTT   TTATCATCAT
   TATTAGCTTA   CTTTCATAAT   TGCGACTGGT   TCCAATTGAC
   AAGCTTTTGA   TTTTAACGAC   TTTTAACGAC   AACTTGAGAA
   GATCAAAAAA   CAACTAATTA   TTCGAAACG-3'
```

and functional equivalents thereof.

2. A DNA fragment in accordance with claim 1 further comprising:

a polypeptide coding region; wherein said regulatory region is positioned at the 5' end of said polypeptide coding region.

3. A DNA fragment in accordance with claim 2 further comprising a 3' sequence of DNA downstream of the polypeptide coding region; wherein said 3' sequence of DNA is capable of controlling the polyadenylation, termination of transcription and termination of translation of messenger RNA coded for by said polypeptide coding region.

4. A DNA fragment in accordance with claim 2 wherein said DNA fragment further comprises one or more additional DNA sequences derived from the group consisting of

bacterial plasmid DNA,

bacteriophage DNA,

yeast plasmid DNA, and

yeast chromosomal DNA.

5. A DNA fragment in accordance with claim 4 wherein said yeast chromosomal DNA comprises an autonomously replicating DNA sequence and a marker gene.

6. A DNA fragment in accordance with claim 5 wherein said fragment is in the form of a closed circular hybrid plasmid.

7. A transformed yeast strain wherein yeast strain is a host for the DNA fragment of claim 2.

8. A transformed yeast strain wherein said yeast strain is a host for the DNA fragment of claim 3.

9. A transformed yeast strain wherein said yeast strain is a host for the DNA fragment of claim 6.

10. A DNA fragment responsive to the presence of methanol in the culture medium in which the host bearing said fragment is grown; wherein said fragment is incorporated into the genome of said host; and wherein said fragment comprises at least the nucleotide sequence:

```
                          5'-ATA      ATCTCATTAA    TGCTTAGCGC
          AGTCTCTCTA      TCGCTTCTGA  ACCCGGTGGC    ACCTGTGCCG
          AAACGCAAAT      GGGGAAACAA  CCCGCTTTTT    GGATGATTAT
          GCATTGTCCT      CCACATTGTA  TGCTTCCAAG    ATTCTGGTGG
          GAATACTGCT      GATAGCCTAA  CGTTCATGAT    CAAAATTTAA
          CTGTTCTAAC      CCCTACTTGG  ACAGGCAATA    TATAAACAGA
          AGGAAGCTGC      CCTGTCTTAA  ACCTTTTTTT    TTATCATCAT
          TATTAGCTTA      CTTTCATAAT  TGCGACTGGT    TCCAATTGAC
          AAGCTTTTGA      TTTTAACGAC  TTTTAACGAC    AACTTGAGAA
          GATCAAAAAA      CAACTAATTA  TTCGAAACG-3'
```

and functional equivalents thereof.

11. A DNA fragment in accordance with claim 10 further comprising:

a polypeptide coding region; wherein said regulatory region is positioned at the 5' end of said polypeptide coding region.

12. A DNA fragment in accordance with claim 11 further comprising a 3' sequence of DNA downstream of the polypeptide coding region; wherein said 3' sequence of DNA is capable of controlling the polyadenylation, termination of transcription and termination of translation of messenger RNA coded for by said polypeptide coding region.

13. A DNA fragment in accordance with claim 11 wherein said DNA fragment further comprises one or more additional DNA sequences derived from the group consisting of

bacterial plasmid DNA,

bacteriophage DNA,

yeast plasmid DNA, and

yeast chromosomal DNA.

14. A DNA fragment in accordance with claim 13 wherein said yeast chromosomal DNA comprises a marker gene.

15. A DNA fragment in accordance with claim 13 wherein said yeast chromosomal DNA comprises an autonomously replicating DNA sequence.

16

16. A DNA fragment in accordance with claim 13 wherein said fragment is in the form of a closed circular hybrid plasmid prior to integration into the genome of the host.

17. A DNA fragment in accordance with claim 13 wherein said fragment is a linear fragment.

18. A transformed yeast strain wherein said yeast strain is a host for the DNA fragment of claim 11.

19. A transformed yeast strain wherein said yeast strain is a host for the DNA fragment of claim 12.

20. A transformed yeast strain wherein said yeast strain is a host for the DNA fragment of claim 17.

21. A DNA fragment having the properties of an upstream activator sequence, wherein said fragment confers on heterologous promoter nucleotide sequences positioned downstream thereof the ability to be induced in the presence of methanol and repressed in the presence of catabolite repressing carbon sources, and wherein said fragment comprises at least the nucleotide sequence:

|             | 5'-ATA     | ATCTCATTAA | TGCTTAGCGC |
|-------------|------------|------------|------------|
| AGTCTCTCTA  | TCGCTTCTGA | ACCCGGTGGC | ACCTGTGCCG |
| AAACGCAAAT  | GGGGAAACAA | CCCGCTTTTT | GGATGATTAT |
| GCATTGTCCT  | CCACATTGTA | TGCTTCCAAG | ATTCTGGTGG |
| GAATACTGCT  | GATAGCCTAA | CGTTCATGAT | CAA-3'     |

and functional equivalents thereof.

22. A DNA fragment in accordance with claim 21 further comprising:

a polypeptide coding region; wherein said regulatory region is positioned at the 5' end of said polypeptide coding region.

23. A DNA fragment in accordance with claim 22 further comprising a 3' sequence of DNA downstream of the polypeptide coding region; wherein said 3' sequence of DNA is capable of controlling the polyadenylation, termination of transcription and termination of translation of messenger RNA coded for by said polypeptide coding region.

24. A DNA fragment in accordance with claim 22 wherein said DNA fragment further comprises one or more additional DNA sequences derived from the group consisting of

bacterial plasmid DNA,

bacteriophage DNA,

yeast plasmid DNA, and

yeast chromosomal DNA.

25. A DNA fragment in accordance with claim 24 wherein said yeast chromosomal DNA comprises a marker gene.

26. A DNA fragment in accordance with claim 24 wherein said yeast chromosomal DNA comprises an autonomously replicating sequence.

27. A DNA fragment in accordance with claim 24 wherein said fragment is in the form of a closed circular hybrid plasmid prior to integration into the genome of the host.

28. A DNA fragment in accordance with claim 24 wherein said fragment is a linear fragment.

29. A transformed yeast strain wherein said yeast strain is a host for the DNA fragment of claim 22.

30. A transformed yeast strain wherein said yeast strain is a host for the DNA fragment of claim 23.

31. A transformed yeast strain wherein said yeast strain is a host for the DNA fragment of claim 28.

32. A method for controlling the responsiveness of an integrated regulatory region to the presence of methanol which comprises employing as the methanol responsive regulatory region positioned upstream of the coding sequence to be expressed at least the following nucleotides:

|             |             |              | 5'-AATTTAA  |
|-------------|-------------|--------------|------------|
| CTGTTCTAAC  | CCCTACTTGG  | ACAGGCAATA   | TATAAACAGA |
| AGGAAGCTGC  | CCTGTCTTAA  | ACCTTTTTTT   | TTATCATCAT |
| TATTAGCTTA  | CTTTCATAAT  | TGCGACTGGT   | TCCAATTGAC |
| AAGCTTTTGA  | TTTTAACGAC  | TTTTAACGAC   | AACTTGAGAA |
| GATCAAAAAA  | CAACTAATTA  | TTCGAAACG-3' |            |

in order to obtain nominal methanol responsiveness, and incrementally including the following nucleotides upstream thereof to obtain a higher degree of methanol responsiveness:

|  |  | 5'-ATA | ATCTCATTAA | TGCTTAGCGC |
|---|---|---|---|---|
| AGTCTCTCTA | TCGCTTCTGA | ACCCGGTGGC | ACCTGTGCCG |
| AAACGCAAAT | GGGGAAACAA | CCCGCTTTTT | GGATGATTAT |
| GCATTGTCCT | CCACATTGTA | TGCTTCCAAG | ATTCTGGTGG |
| GAATACTGCT | GATAGCCTAA | CGTTCATGAT | CAA-3' |

wherein the maximum degree of methanol response is obtained employing all the above enumerated nucleotides, and wherein varying degrees of methanol response are obtained in accordance with the response table below employing an intermediate number of nucleotides derived from the latter sequence:

| Nucleotide Sequence | % Methanol Induction |
|---|---|
| 0-197 | 5 |
| 0-197 plus 347-372 | 5 |
| 0-197 plus 264-372 | 25 |
| 0-372 | 100 |

33. A method for controlling the responsiveness of an autonomous regulatory region to the presence of methanol which comprises employing as the regulatory region at least the following nucleotides:

|  |  |  | 5'-AATTTAA |
|---|---|---|---|
| CTGTTCTAAC | CCCTACTTGG | ACAGGCAATA | TATAAACAGA |
| AGGAAGCTGC | CCTGTCTTAA | ACCTTTTTTT | TTATCATCAT |
| TATTAGCTTA | CTTTCATAAT | TGCGACTGGT | TCCAATTGAC |
| AAGCTTTTGA | TTTTAACGAC | TTTTAACGAC | AACTTGAGAA |
| GATCAAAAAA | CAACTAATTA | TTCGAAACG-3' |  |

in order to obtain nominal methanol responsiveness, and incrementally including the following nucleotides upstream thereof to obtain a higher degree of methanol responsiveness:

18

|  |  |  |  |
|---|---|---|---|
|  |  | 5'-AGATCTAA | CATCCAAAGA |
| CGAAAGGTTG | AATGAAACCT | TTTTGCCATC | CGACATCCAC |
| AGGTCCATTC | TCACACATAA | GTGCCAAACG | CAACAGGAGG |
| GGATACACTA | GCAGCAGACG | TTGCAAACGC | AGGACTCATC |
| CTCTTCTCTA | ACACCATTTT | GCATGAAAAC | AGCCAGTATT |
| GGGCTTGATG | GAGCTCGCTC | ATTCCAATTC | CTTCTATTAG |
| GCTACTAACA | CCATGACTTT | ATTAGCCTGT | CTATCCTGGC |
| CCCCCTGGCG | AGGTCATGTT | TGTTTATTTC | CGAATGCAAC |
| AAGCTCCGCA | TTACACCCGA | ACATCACTCC | AGATGAGGGC |
| TTTCTGAGTG | TGGGGTCAAA | TAGTTTCATG | TTCCCAAATG |
| GCCCAAAACT | GACAGTTTAA | ACGCTGTCTT | GGAACCTAAT |
| ATGACAAAAG | CGTGATCTCA | TCCAAGATGA | ACTAAGTTTG |
| GTTCGTTGAA | ATGCTAACGG | CCAGTTGGTC | AAAAAGAAAC |
| TTCCAAAAGT | CGCCATACCG | TTTGTCTTGT | TTGGTATTGA |
| TTGACGAATG | CTCAAAAATA | ATCTCATTAA | TGCTTAGCGC |
| AGTCTCTCTA | TCGCTTCTGA | ACCCGGTGGC | ACCTGTGCCG |
| AAACGCAAAT | GGGGAAACAA | CCCGCTTTTT | GGATGATTAT |
| GCATTGTCCT | CCACATTGTA | TGCTTCCAAG | ATTCTGGTGG |
| GAATACTGCT | GATAGCCTAA | CGTTCATGAT | CAA-3' |

wherein the maximum degree of methanol response is obtained employing all the above enumerated nucleotides, and varying degrees of methanol response, in accordance with the table below, are obtained employing an intermediate number of nucleotides derived from the latter sequence:

| Nucleotide Sequence | % Methanol Induction |
|---|---|
| 0-197 | 4 |
| 0-216 | 13 |
| 0-372 | 50 |
| 0-499 | 50 |
| 0-709 | 100 |

**Claims**

1. A DNA fragment responsive to the presence of methanol in the culture medium in which the host bearing said fragment is grown; wherein said fragment is maintained as an autonomous element in said host; and wherein said fragment comprises at least the nucleotide sequence:

```
5'-GCTACTAACA    CCATGACTTT    ATTAGCCTGT    CTATCCTGGC
CCCCCTGGCG      AGGTCATGTT    TGTTTATTTC    CGAATGCAAC
AAGCTCCGCA      TTACACCCGA    ACATCACTCC    AGATGAGGGC
TTTCTGAGTG      TGGGGTCAAA    TAGTTTCATG    TTCCCAAATG
GCCCAAAACT      GACAGTTTAA    ACGCTGTCTT    GGAACCTAAT
ATGACAAAAG      CGTGATCTCA    TCCAAGATGA    ACTAAGTTTG
GTTCGTTGAA      ATGCTAACGG    CCAGTTGGTC    AAAAAGAAAC
TTCCAAAAGT      CGCCATACCG    TTTGTCTTGT    TTGGTATTGA
TTGACGAATG      CTCAAAAATA    ATCTCATTAA    TGCTTAGCGC
AGTCTCTCTA      TCGCTTCTGA    ACCCGGTGGC    ACCTGTGCCG
AAACGCAAAT      GGGGAAACAA    CCCGCTTTTT    GGATGATTAT
GCATTGTCCT      CCACATTGTA    TGCTTCCAAG    ATTCTGGTGG
GAATACTGCT      GATAGCCTAA    CGTTCATGAT    CAAAATTTAA
CTGTTCTAAC      CCCTACTTGG    ACAGGCAATA    TATAAACAGA
AGGAAGCTGC      CCTGTCTTAA    ACCTTTTTTT    TTATCATCAT
TATTAGCTTA      CTTTCATAAT    TGCGACTGGT    TCCAATTGAC
AAGCTTTTGA      TTTTAACGAC    TTTTAACGAC    AACTTGAGAA    -
GATCAAAAAA      CAACTAATTA    TTCGAAACG⁻3'
```

and functional equivalents thereof.

2. A DNA fragment responsive to the presence of methanol in the culture medium in which the host bearing said fragment is grown; wherein said fragment is incorporated into the genome of said host; and wherein said fragment comprises at least the nucleotide sequence:

```
                        5'-ATA        ATCTCATTAA    TGCTTAGCGC
AGTCTCTCTA      TCGCTTCTGA    ACCCGGTGGC    ACCTGTGCCG
AAACGCAAAT      GGGGAAACAA    CCCGCTTTTT    GGATGATTAT
GCATTGTCCT      CCACATTGTA    TGCTTCCAAG    ATTCTGGTGG
GAATACTGCT      GATAGCCTAA    CGTTCATGAT    CAAAATTTAA
CTGTTCTAAC      CCCTACTTGG    ACAGGCAATA    TATAAACAGA
AGGAAGCTGC      CCTGTCTTAA    ACCTTTTTTT    TTATCATCAT
TATTAGCTTA      CTTTCATAAT    TGCGACTGGT    TCCAATTGAC
AAGCTTTTGA      TTTTAACGAC    TTTTAACGAC    AACTTGAGAA
GATCAAAAAA      CAACTAATTA    TTCGAAACG-3'
```

and functional equivalents thereof.

3. A DNA fragment having the properties of an upstream activator sequence, wherein said fragment confers on heterologous promoter nucleotide sequence positioned downstream thereof the ability to be induced in the presence of methanol and repressed in the presence of catabolite repressing carbon sources, and wherein said fragment comprises at least the nucleotide sequence:

|            |            | 5'-ATA     | ATCTCATTAA | TGCTTAGCGC |
|------------|------------|------------|------------|------------|
|            | AGTCTCTCTA | TCGCTTCTGA | ACCCGGTGGC | ACCTGTGCCG |
|            | AAACGCAAAT | GGGGAAACAA | CCCGCTTTTT | GGATGATTAT |
|            | GCATTGTCCT | CCACATTGTA | TGCTTCCAAG | ATTCTGGTGG |
|            | GAATACTGCT | GATAGCCTAA | CGTTCATGAT | CAA-3'      |

and functional equivalents thereof.

4. The DNA fragment of any of claims 1 - 3, further comprising: a polypeptide coding region; wherein said regulatory region is positioned at the 5′ end of said polypeptide coding region.

5. The DNA fragment of claim 4 further comprising a 3′ sequence of DNA downstream of the polypeptide coding region; wherein said 3′ sequence of DNA is capable of controlling the polyadenylation, termination of transcription and termination of translation of messenger RNA coded for by said polypeptide coding region.

6. The DNA fragment of claim 4 characterized in that said DNA fragment further comprises one or more additional DNA sequences derived from the group consisting of
bacterial plasmid DNA,
bacteriophage DNA,
yeast plasmid DNA,and
yeast chromosomal DNA.

7. The DNA fragment of claim 1 and 6 characterized in that said yeast chromosomal DNA comprises an autonomously replicating DNA sequence and a marker gene; in particular wherein said fragment is in the form of a closed circular hybrid plasmid.

8. The DNA fragment of claim 2 and 6 or claim 3 and 6 characterized in that said yeast chromosomal DNA comprises a marker gene; in particular wherein said yeast chromosomal DNA comprises an autonomously replicating DNA sequence; in particular wherein said fragment is in the form of a closed circular hybrid plasmid prior to integration into the genome of the host; in particular wherein said fragment is a linear fragment.

9. A transformed yeast strain wherein said yeast strain is a host for the DNA fragment of any of claims 4, 5, 7 or 8.

10. A method for controlling the responsiveness of an integrated regulatory region to the presence of methanol which comprises employing as the methanol responsive regulatory region positioned upstream of the coding sequence to be expressed at least the following nucleotides:

|            |            |            |            | 5'-AATTTAA |
|------------|------------|------------|------------|------------|
|            | CTGTTCTAAC | CCCTACTTGG | ACAGGCAATA | TATAAACAGA |
|            | AGGAAGCTGC | CCTGTCTTAA | ACCTTTTTTT | TTATCATCAT |
|            | TATTAGCTTA | CTTTCATAAT | TGCGACTGGT | TCCAATTGAC |
|            | AAGCTTTTGA | TTTTAACGAC | TTTTAACGAC | AACTTGAGAA |
|            | GATCAAAAAA | CAACTAATTA | TTCGAAACG-3' |            |

in order to obtain nominal methanol responsiveness, and incrementally including the following nucleotides upstream thereof to obtain a higher degree of methanol responsiveness:

|            |            | 5'-ATA     | ATCTCATTAA | TGCTTAGCGC |
|------------|------------|------------|------------|------------|
|            | AGTCTCTCTA | TCGCTTCTGA | ACCCGGTGGC | ACCTGTGCCG |
|            | AAACGCAAAT | GGGGAAACAA | CCCGCTTTTT | GGATGATTAT |
|            | GCATTGTCCT | CCACATTGTA | TGCTTCCAAG | ATTCTGGTGG |
|            | GAATACTGCT | GATAGCCTAA | CGTTCATGAT | CAA-3'      |

21

wherein the maximum degree of methanol response is obtained employing all the above enumerated nucleotides, and wherein varying degrees of methanol response are obtained in accordance with the response table below employing an intermediate number of nucleotides derived from the latter sequence:

| Nucleotide Sequence | % Methanol Induction |
|---|---|
| 0-197 | 5 |
| 0-197 plus 347-372 | 5 |
| 0-197 plus 264-372 | 25 |
| 0-372 | 100 |

11. A method for controlling the responsiveness of an autonomous regulatory region to the presence of methanol which comprises employing as the regulatory region at least the following nucleotides:

```
                                                      5'-AATTTAA
        CTGTTCTAAC    CCCTACTTGG    ACAGGCAATA    TATAAACAGA
        AGGAAGCTGC    CCTGTCTTAA    ACCTTTTTTT    TTATCATCAT
        TATTAGCTTA    CTTTCATAAT    TGCGACTGGT    TCCAATTGAC
        AAGCTTTTGA    TTTTAACGAC    TTTTAACGAC    AACTTGAGAA
        GATCAAAAAA    CAACTAATTA    TTCGAAACG-3'
```

in order to obtain nominal methanol responsiveness, and incrementally including the following nucleotides upstream thereof to obtain a higher degree of methanol responsiveness:

22

|  |  | 5'-AGATCTAA | CATCCAAAGA |
|---|---|---|---|
| CGAAAGGTTG | AATGAAACCT | TTTTGCCATC | CGACATCCAC |
| AGGTCCATTC | TCACACATAA | GTGCCAAACG | CAACAGGAGG |
| GGATACACTA | GCAGCAGACG | TTGCAAACGC | AGGACTCATC |
| CTCTTCTCTA | ACACCATTTT | GCATGAAAAC | AGCCAGTATT |
| GGGCTTGATG | GAGCTCGCTC | ATTCCAATTC | CTTCTATTAG |
| GCTACTAACA | CCATGACTTT | ATTAGCCTGT | CTATCCTGGC |
| CCCCCTGGCG | AGGTCATGTT | TGTTTATTTC | CGAATGCAAC |
| AAGCTCCGCA | TTACACCCGA | ACATCACTCC | AGATGAGGGC |
| TTTCTGAGTG | TGGGGTCAAA | TAGTTTCATG | TTCCCAAATG |
| GCCCAAAACT | GACAGTTTAA | ACGCTGTCTT | GGAACCTAAT |
| ATGACAAAAG | CGTGATCTCA | TCCAAGATGA | ACTAAGTTTG |
| GTTCGTTGAA | ATGCTAACGG | CCAGTTGGTC | AAAAAGAAAC |
| TTCCAAAAGT | CGCCATACCG | TTTGTCTTGT | TTGGTATTGA |
| TTGACGAATG | CTCAAAAATA | ATCTCATTAA | TGCTTAGCGC |
| AGTCTCTCTA | TCGCTTCTGA | ACCCGGTGGC | ACCTGTGCCG |
| AAACGCAAAT | GGGGAAACAA | CCCGCTTTTT | GGATGATTAT |
| GCATTGTCCT | CCACATTGTA | TGCTTCCAAG | ATTCTGGTGG |
| GAATACTGCT | GATAGCCTAA | CGTTCATGAT | CAA-3' |

wherein the maximum degree of methanol response is obtained employing all the above enumerated nucleotides, and varying degrees of methanol response, in accordance with the table below, are obtained employing an intermediate number of nucleotides derived from the latter sequence:

| Nucleotide Sequence | % Methanol Induction |
|---|---|
| 0-197 | 4 |
| 0-216 | 13 |
| 0-372 | 50 |
| 0-499 | 50 |
| 0-709 | 100 |

FIG. 1

FIG. 3

FIG. 2

FIG. 4

FIG. 5

0 244 598

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | MOLECULAR AND CELLULAR BIOLOGY, vol. 5, no. 5, May 1985, pages 1111-1121, American Society for Microbiology; S.B. ELLIS et al.: "Isolation of alcohol oxidase and two other methanol regulatable genes from the Yeast Pichia pastoris" <br> * Whole document * | 1-9 | C 12 N 15/00 <br> C 12 N 1/16 // <br> (C 12 N 1/16 <br> C 12 R 1:84 ) |
| A | NUCLEIC ACIDS RESEARCH, vol. 13, no. 9, May 1985, pages 3063-3082, Oxford, GB; A.M. LEDEBOER et al.: "Molecular cloning and characterization of a gene coding for methanol oxidase in Hansenula polymorpha" | 1 | |
| P,Y | EP-A-0 183 071 (PHILLIPS PETROLEUM) <br> * Claims * | 6-9 | **TECHNICAL FIELDS SEARCHED (Int. Cl.4)** <br><br> C 12 N |
| A | | 1-5 | |
| P,Y | EP-A-0 180 899 (PHILLIPS PETROLEUM) <br> * Claims * | 6-9 | |

The present search report has been drawn up for all claims

| Place of search | Date of completion of the search | Examiner |
|---|---|---|
| THE HAGUE | 07-07-1987 | DELANGHE L.L.M. |